# EUROPÄISCHE PATENTSCHRIFT

(11) **EP 1 051 119 B1**
(45) Veröffentlichungstag und Bekanntmachung des Hinweises auf die Patenterteilung: **05.01.2005**
(21) Anmeldenummer: 99904816.8
(22) Anmeldetag: 27.01.1999
(51) Int. Cl.: A61B 18/00

(54) **INSTRUMENT ZUM APPLIZIEREN VON LICHT, INSBESONDERE LASERLICHT, IM MENSCHLICHEN ODER TIERISCHEN KÖRPER**
INSTRUMENT FOR APPLYING LIGHT, IN PARTICULAR LASER LIGHT, TO THE HUMAN OR ANIMAL BODY
INSTRUMENT PERMETTANT D'APPLIQUER UNE LUMIERE, NOTAMMENT UNE LUMIERE LASER DANS UN ORGANISME HUMAIN OU ANIMAL

(30) Priorität: 27.01.1998 DE 29801223 U
(43) Veröffentlichungstag der Anmeldung: 15.11.2000
(73) Patentinhaber: Karl Storz GmbH & Co. KG, 78532 Tuttlingen (DE); Sroka, Ronald, 81377 München (DE); Rösler, Peter, 81377 München (DE)
(72) Erfinder: Sroka, Ronald, Laser-Forschungslabor, 81377 München (DE); Rösler, Peter, Laser-Forschungslabor, 81377 München (DE); LEUNIG, Andreas, D-82031 Grünwald (DE)
(74) Vertreter: Heuckeroth, Volker, Dr.
(86) Internationale Anmeldenummer: PCT/EP1999/000509
(87) Internationale Veröffentlichungsnummer: WO 1999/037224

(56) Entgegenhaltungen:
- DE-A- 2 852 653
- DE-A- 2 945 080
- DE-U- 9 109 933
- US-A- 4 881 524
- US-A- 5 209 747
- US-A- 5 222 174
- US-A- 5 267 996
- US-A- 5 281 214

## Beschreibung

Die Erfindung betrifft ein Instrument zum Applizieren von Licht, insbesondere Laserlicht, im menschlichen oder tierischen Körper, insbesondere für die endonasale Laserchirurgie.

Auf dem medizinischen Gebiet der Hals-, Nasen- und Ohrenheilkunde müssen häufig Patienten behandelt werden, deren Nasenatmung behindert ist. Häufigste Ursache für die Behinderung der Nasenatmung sind pathologische Vergrößerungen der oberen, mittleren und/oder unteren Nasenmuschel in der inneren Nase. Das Krankheitsbild pathologisch vergrößerter Nasenmuscheln wird in die Nasenmuschelhypertrophie bzw. die Nasenmuschelhyperplasie unterschieden.

Während die Nasenmuschelhypertrophie eine Volumenzunahme darstellt, die durch eine zelluläre Schwellung, bedingt durch Allergie oder Veranlagung, hervorgerufen wird, also keine echte Gewebszunahme darstellt, handelt es sich bei der Nasenmuschelhyperplasie um eine gutartige, durch den Körper initiierte Zellvermehrung, die eine echte Gewebszunahme darstellt. In beiden Fällen ist es das Ziel der Behandlung, die Nasenatmung durch Verkleinerung der Nasenmuscheln zu verbessern.

Vor dem Hintergrund der technischen Entwicklung wurden verschiedene Therapieverfahren entwickelt. Zu den herkömmlichen Therapieverfahren gehören die Diathermie, beispielsweise die Hochfrequenzwärmetherapie oder die Elektrokoagulation und die Conchotomie, bei der mittels eines mechanischen Schneidinstruments ein Teil der Nasenmuschel weggeschnitten wird.

Bei der Diathermie hat sich als nachteilig herausgestellt, daß die Tiefenwirkung der Wärmebehandlung schlecht steuerbar ist. Außerdem kann es zu einer großflächigen Schädigung der Nasenschleimhaut (Mukosa) kommen. In der frühen postoperativen Phase treten weitere Nebeneffekte auf, z.B. ein Krankheitsgefühl und ein gestörter Atemfluß.

Bei der Conchotomie ist nachteilig, daß diese Therapie nur unter Vollnarkose durchgeführt werden kann, daß starke intraoperative Blutungen hervorgerufen werden und der Gebrauch von Tamponaten wegen der Nachblutungsgefahr erforderlich ist. Außerdem bedingt die Konchotomie eine stationäre Aufnahme von vier bis sieben Tagen und eine lange Rekonvaleszenzzeit. Außerdem besteht die Gefahr von Verwachsungen und Infektionen.

Eine nicht mit den zuvor genannten Nachteilen behaftete Therapieart, die in anderen medizinischen Bereichen erfolgreich angewandt wurde, besteht in der Behandlung pathologischer Gewebsbereiche mittels Laserlicht. Eine Therapie mittels Laserlicht ermöglicht im Unterschied zu den zuvor genannten herkömmlichen Operationsmethoden eine minimal invasive Operation. Auf die möglichen Vorteile einer laserlichtgestützten Therapie der Nasenmuscheln wird bereits in dem in der DE-Fachzeitschrift "HNO aktuell", 1997, Seiten 223 bis 230 veröffentlichten Aufsatz "Laser in der Rhinologie", hingewiesen. Eine tatsächliche Anwendung einer Lasertherapie zur Behandlung der Nasenmuscheln scheiterte bislang jedoch an der Verfügbarkeit eines geeigneten Instruments.

Die Wirkung von Laserlicht auf Gewebe allgemein sind die Koagulation, d.h. eine Erwärmung, bei der Gewebeeiweiß denaturiert wird, und die Ablation und die Vaporisation, bei der Gewebe abgetragen bzw. verdampft wird.

Die Wirkung ist dabei abhängig vom Abstand der Lichtwellenleiterspitze zum Gewebe, durch den die applizierte Energiedichte verändert werden kann. Es haben sich dabei das "Kontaktverfahren" und das "Non-Kontakt-Verfahren" herauskristallisiert. Beim Kontaktverfahren berührt die Lichtwellenleiter-Spitze das Gewebe, wodurch vaporisierende, schneidende Effekte erzielt werden, während im Non-Kontakt-Verfahren zwischen der Lichtwellenleiter-Spitze und dem zu behandelnden Gewebe ein definierter Abstand besteht, wodurch eine Applation bzw. Koagulation von Gewebe stattfindet.

Die Vorteile einer lasergestützten Therapie sind zahlreich, unter anderem ist das Blutungsrisiko wesentlich verringert, die Operationszeit wesentlich verkürzt, es ist keine Vollnarkose erforderlich, sondern lediglich eine Lokalnarkose, und postoperative Komplikationen treten nur mit einer äußerst geringen Wahrscheinlichkeit auf.

Zur Anwendung einer Laserlicht-Therapie zur Verkleinerung der unteren Nasenmuschel werden derzeit versuchsweise vorwiegend Instrumente verwendet, die sich bereits in der Orthopädie bei der Laser-Arthroskopie etabliert haben. Diese Instrumente bestehen aus einem einfachen, gebogenen Rohr, welches den Lichtwellenleiter in sich aufnimmt. Das Rohr bzw. der Rohrschaft ist im Winkel bis zu 30° vorgebogen oder wird nach Einführen des Lichtwellenleiters entsprechend der gegebenen Anatomie des Patienten zurecht gebogen.

Diese bekannten Instrumente weisen jedoch mehrere Nachteile auf.

Beim Einführen des Lichtwellenleiters kann die Spitze und insbesondere deren lichtaustrittsseitige empfindliche geschliffene Stirnfläche beim Durchführen durch die gebogene Stelle des Rohres beschädigt werden. Die lichtaustrittsseitige Stirnfläche des Lichtwellenleiters kann dabei splittern. Die Folge einer so beschädigten Lichtaustrittsfläche des Lichtwellenleiters ist, daß das Laserlicht unkontrolliert seitlich abgestrahlt wird. Dabei besteht die Gefahr, daß nicht beteiligtes Gewebe durch unkontrolliert auch austretendes Laserlicht beschädigt wird, der Patient mithin verletzt wird. Außerdem besteht eine Verletzungsgefahr für den behandelnden Arzt durch das unkontrolliert austretende Laserlicht.

Ein weiterer Nachteil dieser bekannten Instrumente besteht darin, daß ein solches vorgebogenes Rohr nur einen festen Arbeitswinkel, d.h. eine feste Abstrahlrichtung des Laserlichts zuläßt, die für die individuelle Anatomie des jeweiligen Patienten unzureichend ist. Dadurch, daß die Abstrahlrichtung des Laserlichts durch ein Biegen des Rohrs eingestellt wird, sind die Rohre durch Materialermüdung bedingt nicht dauerhaft verwendbar.

Ein weiterer Nachteil dieser bekannten Instrumente besteht darin, daß die einmal getroffene Einstellung der Abstrahlrichtung des Laserlichts während der Behandlung nicht variiert werden kann. Zum Verstellen der Abstrahlrichtung muß das Rohr aus dem Operationsfeld herausgenommen werden und wieder entsprechend gebogen werden, wodurch sich die Operationszeit wesentlich verlängert und die Handhabung sich somit umständlich darstellt.

Die bekannten Instrumente eignen sich somit nicht für eine Applikation von Laserlicht zur Verkleinerung der Nasenmuscheln. Die Erfindung ist jedoch nicht auf dieses zuvor beschriebene Anwendungsgebiet beschränkt, sondern ist grundsätzlich in allen medizinischen Gebieten anwendbar, bei denen ein Lichtwellenleiter in kleine Körperhohlräume bzw. -gänge eingeführt und Therapielicht gezielt mit Punktgenauigkeit auf zu behandelndes Gewebe gerichtet werden soll, insbesondere dann, wenn die Platzverhältnisse sehr beengt sind. Als Beispiele sind u.a. die Arthroskopie, Urologie, Laparoskopie, Neurochirurgie usw. zu nennen.

Aus der US-A-4 881 524 ist ein Instrument zum Applizieren von Licht bekannt, das einen Rohrschaft aufweist, in dessen Inneres ein flexibler Lichtwellenleiter einführbar ist. Ein lichtaustrittsseitiges Ende des Lichtwellenleiters kommt in einem distalen Endabschnitt des Rohrschafts zu liegen, wobei der distale Endabschnitt des Rohrschafts über ein scharnierartiges Gelenk mit dem übrigen Abschnitt des Rohrschafts verbunden ist, so daß der distale Endabschnitt aus einer Längsachse des Rohrschafts heraus verschwenkbar ist. Am proximalen Ende des Rohrschafts ist eine Handhabungseinrichtung zum Verschwenken des distalen Endabschnitts angeordnet, die zumindest ein bewegliches Bedienelement aufweist, das über ein axial bewegliches Kraftübertragungselement kraftschlüssig mit dem distalen Endabschnitt verbunden ist. Der distale Endabschnitt läßt sich aus der Längsachse des Rohrschafts nur zu einer Seite hin verschwenken.

Ferner ist aus der US-A-5 307 803 ein flexibles Endoskop bekannt. Ein solches flexibles Endoskop weist einen flexiblen Schaft auf, dessen distales Ende flexibel mit dem übrigen Abschnitt des Rohrschafts verbunden ist. Über einen Auslenkmechanismus kann das distale Ende aus der Längsachse in entgegengesetzten Schwenkrichtungen aus der Längsachse heraus verschwenkt werden.

Die Erfindung ist ferner jedoch nicht nur auf medizinische Anwendungen beschränkt, sondern läßt sich auch in technischen Anwendungen verwenden.

Der Erfindung liegt daher die Aufgabe zugrunde, ein Instrument der eingangs genannten Art dahingehend weiterzubilden, daß die vorstehend genannten Nachteile vermieden werden, daß mit dem Instrument ein Lichtwellenleiter zum Applizieren von Licht, insbesondere Laserlicht, gezielt an eine schwer zugängliche Stelle gebracht werden kann, wobei dann ein Abstrahlen des Lichts bei leichter Bedienbarkeit in mehreren Richtungen ermöglicht werden soll.

Erfindungsgemäß wird diese Aufgabe durch ein Instrument zum Applizieren von Licht, insbesondere Laserlicht im menschlichen oder tierischen Körper, insbesondere für die endonasale Laserchirurgie, gelöst, mit einem Rohrschaft, in dessen Inneres ein flexibler Lichtwellenleiter einführbar ist, wobei ein lichtaustrittsseitiges Ende des Lichtwellenleiters in einem distalen Endabschnitt des Rohrschafts zu liegen kommt, wobei der distale Endabschnitt des Rohrschafts über ein scharnierartiges Gelenk mit dem übrigen Abschnitt des Rohrschafts verbunden ist, so daß der distale Endabschnitt aus einer Längsachse des Rohrschafts heraus verschwenkbar ist, und wobei am proximalen Ende des Rohrschafts eine Handhabungseinrichtung zum Verschwenken des distalen Endabschnitts angeordnet ist, die zumindest ein bewegliches Bedienelement aufweist, das über ein axial bewegliches Kraftübertragungselement kraftschlüssig mit dem distalen Endabschnitt verbunden ist, wobei der distale Endabschnitt durch Betätigen des beweglichen Bedienelements in entgegengesetzten Schwenkrichtungen in einem Winkelbereich zwischen einem Winkel -|α₁| und einem Winkel +|α₂| aus der Längsachse des Rohrschafts heraus verschwenkbar ist.

Im Unterschied zu den aus dem Stand der Technik bekannten Instrumenten, die ein flexibles Rohr bzw. einen flexiblen Rohrschaft aufweisen, ist bei dem erfindungsgemäßen Instrument vorgesehen, den distalen Endabschnitt, in dem das lichtaustrittsseitige Ende des Lichtwellenleiters bzw. dessen Spitze zu liegen kommt, gelenkig mit dem übrigen Abschnitt des Rohrschafts zu verbinden. Der Rohrschaft ist demnach zumindest zweiteilig ausgebildet, wobei die beiden Teile, der distale Endabschnitt und der übrige Abschnitt des Rohrschafts, eine Gelenkverbindung aufweisen. Der distale Endabschnitt ist somit um eine definierte Schwenkachse aus der Längsachse des Rohrschafts heraus verschwenkbar. Dadurch wird zunächst als wesentlicher Vorteil erreicht, daß der Lichtwellenleiter in den Rohrschaft einführbar ist, wenn der distale Endabschnitt mit dem übrigen Abschnitt des Rohrschafts geradlinig fluchtend ausgerichtet ist. In dieser 0°-Stellung des distalen Endabschnitts kann der Lichtwellenleiter somit ohne die Gefahr einer Beschädigung der lichtaustrittsseitigen Stirnfläche bis zum äußersten distalen Ende des Rohrschafts vorgeschoben werden. Der Rohrschaft mit dem darin eingeführten Lichtwellenleiter kann dann beispielsweise zur Verwendung in der endonasalen Laserchirurgie in die Nase eingeführt werden. Durch Betätigen des zumindest einen beweglichen Bedienelements der Handhabungseinrichtung kann dann der distale Endabschnitt des Rohrschaftes und damit das darin befindliche lichtaustrittsseitige Ende des Lichtwellenleiters um die definierte Schwenkachse verschwenkt werden, um den Laserlichtstrahl mit hoher Genauigkeit gezielt auf das zu behandelnde Gewebeareal zu richten. Ohne daß das Instrument aus dem Operationsgebiet entnommen werden muß, kann der behandelnde Arzt die Abstrahlrichtung des Laserlichtstrahles verändern, wodurch die Bedienung des erfindungsgemäßen Instruments wesentlich vereinfacht ist. Durch die gelenkige Verbindung des distalen Endabschnittes mit dem übrigen Abschnitt des Rohrschaftes kann eine Materialermüdung durch Biegen wie bei den bekannten Instrumenten nicht auftreten. Das erfindungsgemäße Instrument ermöglicht somit eine sichere gezielte Führung des Laserlichtstrahls im Operationsgebiet ohne die Gefahr einer Beschädigung bzw. Verletzung nicht beteiligter Gewebepartien. Im Falle der Anwendung der Laserlichttherapie zur Verkleinerung der Nasenmuscheln tritt durch das Verdampfen und Abtragen von Gewebe postoperativ eine unmittelbare Atmungserleichterung auf. Durch die Abheilung in der Folgezeit des koagulierten Gewebes wird weiteres Gewebe abgestoßen, was zusätzlich den Atmungsquerschnitt in der inneren Nase vergrößert.

Durch das vorgesehene Scharniergelenk wird eine besonders stabile gelenkige Verbindung zwischen dem distalen Endabschnitt und dem übrigen Rohrschaft geschaffen, weil keine auf Biegung beanspruchten Teile verwendet werden müssen. Ein weiterer Vorteil besteht darin, daß sich der distale Endabschnitt des Rohrschaftes und damit die Abstrahlrichtung des Lichts zu beiden Seiten der Längsachse des Rohrschafts abwinkeln läßt, wenn sowohl α₁ als auch α₂ von null verschieden gewählt sind, ohne daß dazu die Lage des gesamten Instruments, insbesondere die Lage der Handhabungseinrichtung, durch den Arzt verändert werden muß. Der Winkel - α₁ kann dabei 0 bis - 90° oder mehr und der Winkel + α₂ ebenfalls 0 bis + 90° oder mehr betragen, wobei die maximalen Winkel durch die maximale Biegbarkeit des Lichtwellenleiters einerseits und durch die Bedingungen der Totalreflektion zur Leitung des Lichts in dem Lichtwellenleiter andererseits begrenzt sind.

In einer bevorzugten Ausgestaltung ist der Rohrschaft als Ganzes oder zumindest ein Abschnitt des Rohrschafts, an dem der distale Endabschnitt befestigt ist, bezüglich der Handhabungseinrichtung um die Längsachse des Rohrschafts drehbar.

Durch die Drehbarkeit des Rohrschafts wird in Verbindung mit der Verschwenkbarkeit des distalen Endabschnitts des Rohrschafts eine Abwinklung der Lichtabstrahlung über einen dreidimensionalen Raumwinkelbereich ermöglicht und somit die Variabilität des Instruments noch weiter vergrößert. Zum Verdrehen des Rohrschaftes kann dazu an der Handhabungseinrichtung ein Stellrad vorgesehen sein, daß eine leichte Verdrehung des Rohrschaftes ermöglicht. Ebenso kann der Rohrschaft eine weitere Unterteilung mit einer Drehkupplung aufweisen.

In einer weiteren bevorzugten Ausgestaltung weist der distale Endabschnitt eine axiale Länge auf, die nur geringfügig größer als der Außendurchmesser des übrigen Rohrschafts ist.

Diese Maßnahme ist insbesondere bei der Verwendung des erfindungsgemäßen Instruments in sehr kleinen Operationsgebieten von Vorteil, beispielsweise in schmalen kanalartigen Körpergängen, weil dann eine Verschwenkung des distalen Endabschnittes über einen größeren Winkel nicht durch die beengten Platzverhältnisse im Operationsgebiet behindert wird.

In einer weiteren bevorzugten Ausgestaltung weist der distale Endabschnitt etwa den gleichen Außendurchmesser auf wie der übrige Abschnitt des Rohrschafts.

Hierbei ist von Vorteil, daß beim Einführen des Rohrschaftes in ein Operationsgebiet zwischen dem distalen Endabschnitt und dem übrigen Abschnitt des Rohrschaftes keine radiale Stufe vorhanden ist, die zu einer mechanischen Verletzung von Gewebe führen oder das Einführen des Instruments behindern könnte.

In einer weiteren bevorzugten Ausgestaltung weist der distale Endabschnitt eine Fixierhülse zum Fixieren des lichtaustrittsseitigen Endes des Lichtwellenleiters auf.

Hierdurch wird der Vorteil erzielt, daß das lichtaustrittsseitige Ende des Lichtwellenleiters kein radiales und/oder axiales Spiel in dem distalen Endabschnitt des Rohrschaftes besitzt, welches eine punktgenaue Applikation des Laserlichts stören könnte. Durch diese Ausgestaltung wird somit die Genauigkeit der Führung des Lichtstrahles verbessert.

In einer weiteren bevorzugten Ausgestaltung weist der Rohrschaft einen Anschluß zum Verbinden des Rohrschafts mit einer Saug- und/oder Spüleinrichtung auf, wobei der Innenraum des Rohrschafts einen Saug- und/oder Spülquerschnitt auch bei eingesetztem Lichtwellenleiter aufweist.

Hierbei ist von Vorteil, daß eine Möglichkeit zum Absaugen von bei der Vaporisation von Gewebe entstehender toxisch wirkender oder die Sicht behindernder Rauchgase oder Dämpfe geschaffen wird. Die Gase können somit über den Rohrschaft sicher aus dem Operationsgebiet abgeleitet werden. Durch Einleiten einer Spülflüssigkeit kann das Operationsgebiet außerdem gespült werden, wodurch die Sicht durch ein zusätzlich eingeführtes Endoskop zur endoskopischen Kontrolle der Operation stets erhalten bleibt. Es können für die Absaugung und Spülung auch zwei Anschlüsse vorgesehen sein.

In einer weiteren bevorzugten Ausgestaltung weist der verschwenkbare distale Endabschnitt umfänglich verteilt Öffnung zum Saugen und/oder Spülen auf.

Hierbei ist von Vorteil, daß das Absaugen von Gasen, Flüssigkeiten und Gewebsstücken sowie das Spülen des Operationsgebietes durch die umfänglich verteilten Öffnungen allseitig durch den distalen Endabschnitt erfolgen kann.

In einer weiteren bevorzugten Ausgestaltung ist die Handhabungseinrichtung in Form einer scherengriffartigen Griffanordnung ausgebildet.

Eine solche Handhabungseinrichtung ist besonders vorteilhaft, weil der Arzt an solche Griffanordnungen bereits von medizinischen Zangen, Scheren und dergleichen gewöhnt ist. Das Führen des Laserlichtstrahls im Operationsgebiet kann der Arzt somit genauso sicher handhaben wie beispielsweise das Führen eines Schnittes mit einer medizinischen Zange. Durch diese Ausgestaltung wird mit anderen Worten eine "Laserzange" geschaffen.

In einer weiteren bevorzugten Ausgestaltung ist das Kraftübertragungselement als draht- oder stabförmiges Zug- und Schubelement ausgebildet, das durch das Innere des Rohrschafts verläuft.

Bei dieser Ausgestaltung wird ein konstruktiv einfaches Kraftübertragungselement als kraftschlüssige Verbindung zwischen dem verschwenkbaren distalen Endabschnitt und der Handhabungseinrichtung bereitgestellt. Das draht- oder stabförmige Zug- und Schubelement ist dabei bevorzugt im Innern des Rohrschafts aus angeordnet, um die Außenabmessung des Instruments nicht zu vergrößern.

In einem weiteren bevorzugten Ausführungsbeispiel ist das Kraftübertragungselement als in dem Rohrschaft axial verschieblich angeordneter Innenrohrschaft ausgebildet, wobei der Lichtwellenleiter in das Innere des Innenrohrschaftes einführbar ist.

Diese Ausgestaltung des Kraftübertragungselementes hat den Vorteil, daß es besonders stabil ausgebildet ist und auf Zug und Druck gleichermaßen sicher arbeitet. Diese Ausgestaltung des Kraftübertragungselements führt außerdem zu einer radial besonders klein bauenden Bauweise des Instruments mit einem Doppelrohrschaft. Dadurch, daß der Innenrohrschaft als Führung und Aufnahme für den Lichtwellenleiter ausgebildet ist, läßt sich dieser wiederum besonders leicht einführen. Gegenüber dem zuvor genannten drahtförmigen Zug- und Schubelement wird durch die Ausgestaltung des Kraftübertragungselements als Innenrohrschaft sicher vermieden, daß der Lichtwellenleiter beim Einführen in den Rohrschaft mit diesem unklar kommt und das Einführen erschwert wird. Ein weiterer Vorteil dieser Ausgestaltung besteht darin, daß für den zuvor erwähnten Saug- und/oder Spülquerschnitt im Innern des Rohrschaftes bzw. im Innern des Innenrohrschaftes mehr Platz zur Verfügung steht, so daß nicht nur Gase und Flüssigkeiten, sondern auch kleine Gewebsstücke durch den Rohrschaft abgesaugt werden können.

In einer weiteren bevorzugten Ausgestaltung ist die Verschwenkbarkeit des verschwenkbaren distalen Endabschnitts auf einen vorgegebenen Winkelbereich begrenzt.

Die Begrenzung des Verschwenkbereiches hat den Vorteil, daß die maximale Verschwenkbarkeit des distalen Endabschnittes auf den jeweiligen verwendeten Lichtwellenleiter angepaßt werden kann. Somit wird ein Brechen des Lichtwellenleiters durch ein übermäßiges Biegen vermieden.

In einer weiteren bevorzugten Ausgestaltung ist die Begrenzung des Verschwenkbereiches über einen verstellbaren Anschlag an der Handhabungseinrichtung einstellbar.

Anstatt die Begrenzung des Verschwenkbereiches direkt an der gelenkigen Verbindung des distalen Endabschnittes mit dem übrigen Abschnitt des Rohrschaftes vorzusehen, hat diese Maßnahme den Vorteil, daß das Gelenk, über das der distale Endabschnitt mit dem übrigen Abschnitt des Rohrschafts verbunden ist, so ausgebildet werden kann, daß eine Verschwenkung des distalen Endabschnittes prinzipiell über einen Winkelbereich von beispielsweise etwa - 90° bis + 90° ermöglicht wird. Die Begrenzung der Verschwenkbarkeit des distalen Endabschnittes wird dann durch den Anschlag an der Handhabungseinrichtung begrenzt, indem dieser den Bedienungsweg des zumindest einen beweglichen Bedienelements begrenzt. Durch die Einstellbarkeit des Anschlags kann die Begrenzung der Verschwenkbarkeit des distalen Endabschnittes an den jeweilig verwendeten Lichtwellenleiter angepaßt werden.

In weiteren bevorzugten Ausgestaltung ist der distale Endabschnitt in einer Vielzahl diskreter oder kontinuierlicher Winkelstellungen bezüglich der Längsachse des Rohrschafts arretierbar.

Durch diese Maßnahme wird eine Verstellsicherung für den distalen Endabschnitt geschaffen, der das sichere gezielte Applizieren des Laserlichts weiter verbessert, weil vermieden wird, daß eine definierte eingestellte Abstrahlrichtung des Laserlichtes unerwünscht verstellt werden kann.

Dabei ist es weiterhin bevorzugt, wenn das zumindest eine bewegliche Bedienelement der Handhabungseinrichtung mit einer eine Vielzahl von Raststellungen aufweisenden gezahnten Schiene in Eingriff bringbar ist.

Hierbei ist von Vorteil, daß eine konstruktiv einfache und leicht bedienbare Verstellsicherung für eine Vielzahl von definierten Winkelstellungen des distalen Endabschnitts und damit der Abstrahlrichtung des Laserlichtes geschaffen wird. Wird das bewegliche Bedienelement mit der gezahnten Schiene außer Eingriff gebracht, läßt sich der distale Endabschnitt des Rohrschaftes außerdem kontinuierlich abwinkeln.

Dabei ist es weiterhin bevorzugt, wenn die Zähne der gezahnten Schiene abgerundet sind.

Diese Maßnahme hat den Vorteil, daß sich die Winkelstellung des distalen Endabschnittes auch ohne Lösen der Verstellsicherung in diskreten Stufen variieren läßt, so daß die Handhabung weiter vereinfacht wird, wobei der weitere Vorteil darin besteht, daß die Veränderung der Winkelstellung im wesentlichen ruckfrei erfolgen kann.

In einer weiteren bevorzugten Ausgestaltung ist das distale Ende des verschwenkbaren distalen Endabschnitts abgerundet.

Hierbei ist von Vorteil, daß beim Einführen des Rohrschafts in das Operationsgebiet mechanische Verletzungen von Gewebe vermieden wird, insbesondere im Falle der endonasalen Laserchirurgie die Nasenschleimhaut.

Weitere Vorteile ergeben sich aus der nachfolgenden Beschreibung und der beigefügten Zeichnung.

Es versteht sich, daß die vorstehend genannten und nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ausgewählte Ausführungsbeispiele der Erfindung sind in der Zeichnung dargestellt und werden hiernach mit Bezug auf diese näher beschrieben.

Es zeigen:
- Fig. 1: ein erfindungsgemäßes Instrument zum Applizieren von Licht in Seitenansicht gemäß einem ersten Ausführungsbeispiel;
- Fig. 2: eine teilweise geschnittene Seitenansicht des proximalen Endes des Instruments in Fig. 1;
- Fig. 3: eine stark vergrößerte Darstellung des distalen Endes des Instruments in Fig. 1 in einem Längsschnitt;
- Fig. 4a) bis c): vergrößerte Darstellungen des distalen Endes des Instruments in Fig. 1 in einer Seitenansicht in verschiedenen Winkelstellungen des distalen Endabschnitts;
- Fig. 5: ein weiteres Ausführungsbeispiel eines erfindungsgemäßen Instruments in einer Seitenansicht;
- Fig. 6: das Instrument in Fig. 5 in Draufsicht; und
- Fig. 7: eine vergrößerte Darstellung des distalen Endes des Instruments in Fig. 6.

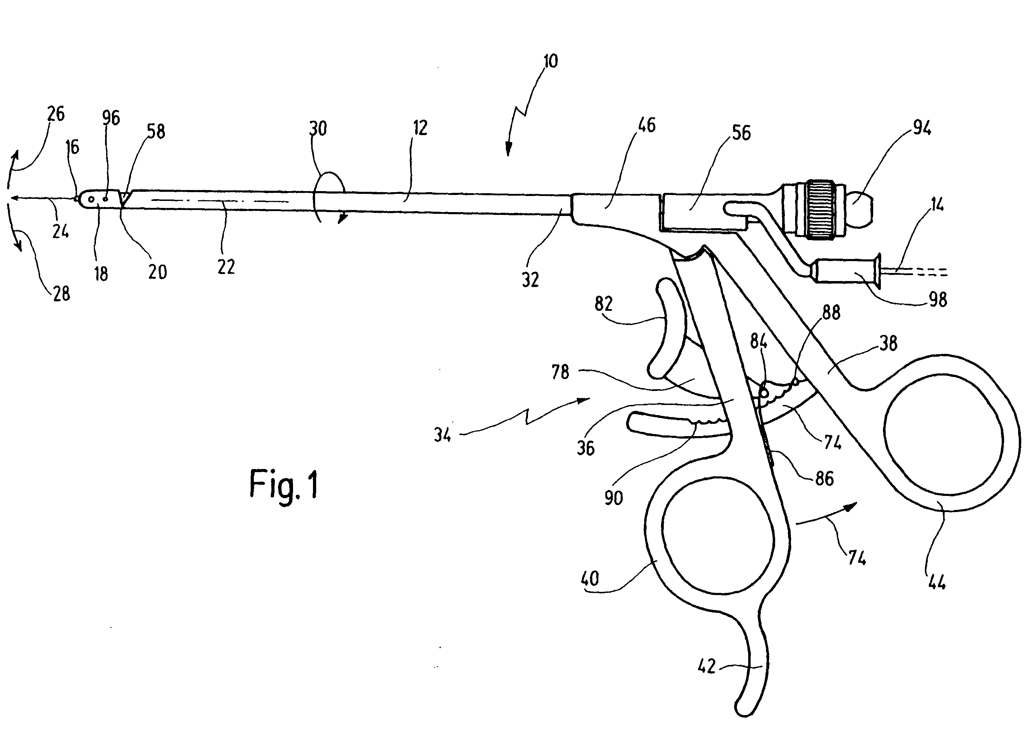

In Fig. 1 ist ein mit dem allgemeinen Bezugszeichen 10 versehenes Instrument zum Applizieren von Licht, insbesondere Laserlicht im menschlichen oder tierischen Körper dargestellt. Das Instrument 10 wird beispielsweise im Rahmen der endonasalen Laserchirurgie zur Verkleinerung pathologisch vergrößerter Nasenmuscheln verwendet.

Das Instrument 10 ist jedoch nicht auf eine derartige Verwendung eingeschränkt, sondern läßt sich auch in anderen medizinischen Gebieten, beispielsweise in der Orthopädie, der Urologie, usw. einsetzen. Das Instrument 10 läßt sich jedoch auch in technischen Anwendungen verwenden. Prinzipiell eignet sich das Instrument 10 zur Verwendung in allen Bereichen, bei denen Licht, insbesondere Laserlicht, gezielt an schwer zugängliche Stellen geführt werden soll.

Das Instrument 10 weist einen lang erstreckten Rohrschaft 12 auf. Der Rohrschaft 12 ist bezüglich seines Außendurchmessers schmal bauend, so daß er bei Verwendung in der endonasalen Laserchirurgie zusammen mit einem Endoskop in die Nase eingeführt werden kann. Der Rohrschaft 12 erstreckt sich ferner gerade.

In den Rohrschaft 12 ist ein flexibler Lichtwellenleiter 14 einführbar, wobei ein distales lichtaustrittsseitiges Ende 16 bzw. eine Spitze des Lichtwellenleiters 14 in einem distalen Endabschnitt 18 des Rohrschafts 12 zu liegen kommt.

Der Lichtwellenleiter 14 ist beispielsweise eine Lichtleitfaser eines Durchmessers, der geringer als 1 mm ist, beispielsweise in Bare- bzw. Sidefirefaser-Ausführung.

Während das lichtaustrittsseitige Ende 16 des Lichtwellenleiters 14 in Fig. 1 als aus dem distalen Endabschnitt 18 des Rohrschaftes 12 distal herausstehend dargestellt ist, kann die Spitze 16 jedoch auch bündig mit dem distalen Ende des distalen Endabschnitts 18 abschließen.

Der distale Endabschnitt 18 des Rohrschafts 12 ist über ein Gelenk 20 mit dem übrigen Abschnitt des Rohrschafts 12 gelenkig verbunden, so daß der distale Endabschnitt 18 aus einer Längsachse 22 des somit zweiteiligen Rohrschafts 12 heraus verschwenkbar ist. Das Gelenk 20 ist ein scharnierartiges Gelenk, daß hiernach noch näher beschrieben wird.

Eine Schwenkachse des Gelenks 20 verläuft dabei quer zur Längsachse 22 des Rohrschafts 12. Durch Verschwenken des distalen Endabschnittes 18 des Rohrschafts 12 läßt sich das lichtaustrittsseitige Ende 16 des Lichtwellenleiters 14 und damit eine Abstrahlrichtung 24 des abgestrahlten Lichtes gemäß Pfeilen 26 bzw. 28 verschwenken. Die Abstrahlrichtung 24 kann somit verschiedene kontinuierlich veränderbare Winkelstellungen bezüglich der Längsachse 22 des Rohrschafts 12 einnehmen.

Der Rohrschaft 12 ist weiterhin um seine Längsachse 22 gemäß einem Pfeil 30 drehbar. Auf diese Weise kann der distale Endabschnitt 18 nicht nur in der Zeichenebene der Fig. 1 verschwenkt werden, sondern durch zusätzliches Drehen des Rohrschaftes 12 auch in allen dazu nicht parallelen Ebenen. Die Abstrahlrichtung 24 kann dadurch in einem Raumwinkelbereich variiert werden.

Zum Verdrehen des Rohrschaftes 12 kann einer mit 32 gekennzeichneten Stelle am Umfang des Rohrschaftes 12 ein Stellring angeordnet sein, der mit dem Rohrschaft 12 drehfest verbunden ist.

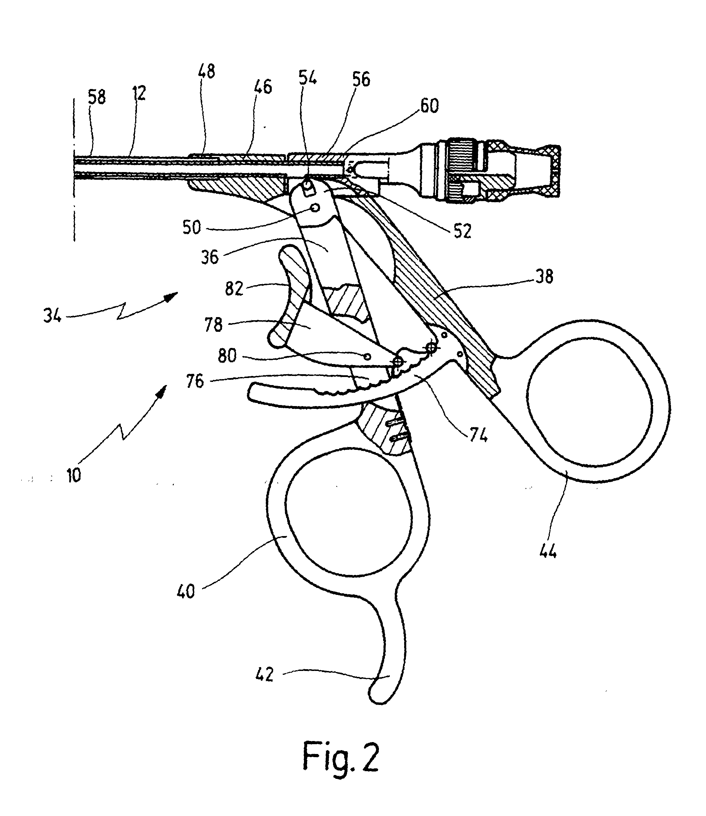

Zum Verschwenken des distalen Endabschnitts 18 weist das Instrument 10 am proximalen Ende des Rohrschafts 12 eine mit dem allgemeinen Bezugszeichen 34 versehene Handhabungseinrichtung auf, die hiernach mit Bezug auf Fig. 1 und 2 näher beschrieben wird.

Die Handhabungseinrichtung 34 ist in Form einer scherengriffartigen Griffanordnung ausgebildet.

Die Handhabungseinrichtung 34 weist ein bewegliches Bedienelement 36 sowie ein unbewegliches Bedienelement 38 auf.

Das bewegliche Bedienelement 36 weist einen Ring 40 zum Durchstecken des Mittelfingers und eine ergonomisch daran angeformte Abstützung 42 für den Ringfinger derselben Hand auf. Das unbewegliche Bedienelement 38 weist einen Ring 44 zum Durchstecken des Daumens auf. Die Griffanordnung aus dem beweglichen Bedienelement 36 und dem unbeweglichen Bedienelement 38 ermöglicht eine besonders ergonomische Handhabung des Instruments 10.

Das unbewegliche Bedienelement 38 weist einen hülsenartigen Abschnitt 46 auf, in dem ein proximales Ende 48 des Rohrschafts 12 um die Längsachse 22 drehbar befestigt ist, so daß der Rohrschaft 12 bezüglich den Bedienelementen 36 und 38 drehbar ist.

Das bewegliche Bedienelement 36 ist mit dem unbeweglichen Bedienelement 38 über ein Drehgelenk 50 gelenkig verbunden.

Ein der Abstützung 42 gegenüber liegendes Ende 52 des beweglichen Bedienelements 36 ist gegabelt ausgeführt und umgreift einen Zapfen 54, der an einem relativ zu dem Rohrschaft 12 axial verschiebbaren Schlitten 56 befestigt ist.

Zur Kraftübertragung von dem beweglichen Bedienelement 36 auf den distalen Endabschnitt 18 des Rohrschaftes 12 ist ein Kraftübertragungselement in Form eines Innenrohrschafts 58 vorgesehen, der in dem Rohrschaft 12 an dessen Innenwand anliegend angeordnet ist. Ein proximales Ende 60 des Innenrohrschafts 58 ist in dem hülsenartigen Schlitten 56 aufgenommen und dort befestigt. Der Innenrohrschaft 58 ist relativ zu dem Rohrschaft 12 axial verschiebbar und erstreckt sich bis zu dem distalen Endabschnitt 18 des Rohrschafts 12.

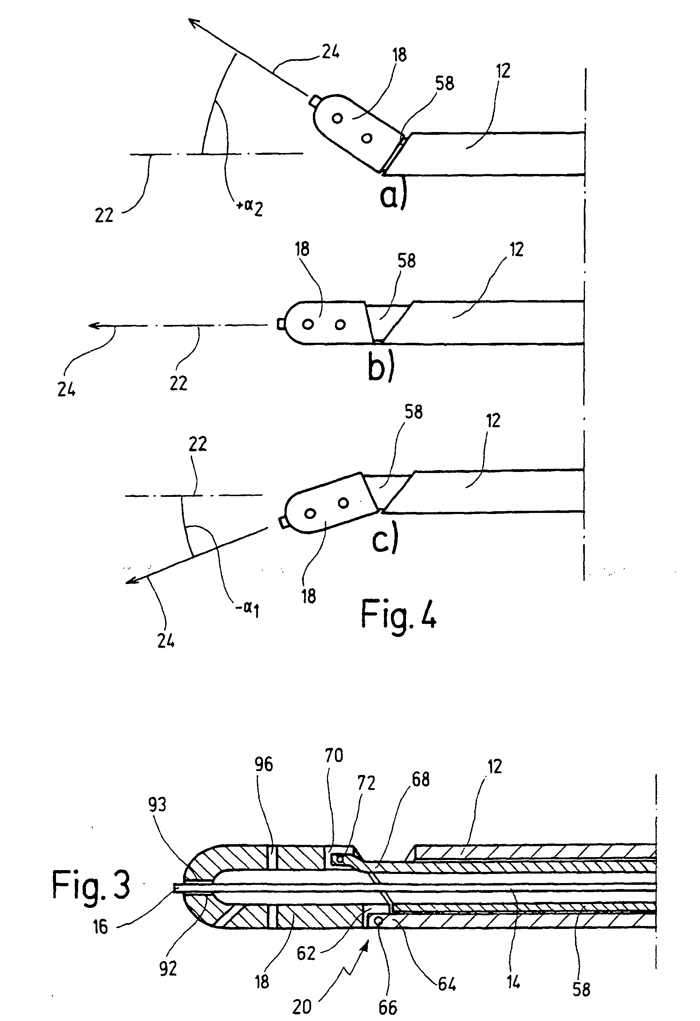

Mit Bezug auf Fig. 3 weist der distale Endabschnitt 18 des Rohrschafts 12 einen proximalen Gabelabschnitt 62 auf, in den ein axialer Fortsatz 64 eingreift, der über einen Gelenkzapfen 66 mit dem Gabelabschnitt 62 des distalen Endabschnitts 18 verbunden ist und das Gelenk 20 bildet.

Das Kraftübertragungselement in Form des Innenrohrschaftes 58 weist einen axialen Fortsatz 68 auf, der in einen dem Gabelabschnitt 62 gegenüberliegenden Gabelabschnitt 70 des distalen Endabschnitts 18 eingreift und mit diesem über einen weiteren Gelenkzapfen 72 gelenkig verbunden ist.

Durch Bewegen des beweglichen Bedienelements 36 in Richtung eines Pfeiles 74 in Fig. 1 wird somit der Schlitten 56 und der mit diesem fest verbundene Innenrohrschaft 58 in Richtung distales Ende verschoben, wodurch der distale Endabschnitt 18 in Richtung des Pfeiles 28 verschwenkt wird. Durch Bewegen des beweglichen Bedienelements 36 entgegen dem Pfeil 74 wird der distale Endabschnitt 18 entsprechend in Richtung des Pfeiles 26 verschwenkt.

Gemäß Fig. a) bis c) kann der distale Endabschnitt 18 und damit die Abstrahlrichtung 24 des Lichtes bezüglich der Längsachse 22 des Rohrschafts 12 Winkelstellungen in einem Bereich zwischen einem Winkel + α₂ und einem Winkel - α₁ einnehmen. Der Winkel + α₂ und der Winkel - α₁ können betragsmäßig gleich groß oder unterschiedlich groß sein. In dem gezeigten Ausführungsbeispiel beträgt der Winkel + α₂ etwa 50°, und der Winkel - α₁ etwa - 10°. Die Erfindung ist jedoch nicht auf diesen Winkelbereich eingeschränkt. Die Maximalwerte von α₁ und α₂ können auch 90° oder mehr betragen, α₁ kann auch 0° sein.

Gemäß Fig. 1 und 2 ist an dem unbeweglichen Bedienelement 38 eine gezahnte Schiene 74 befestigt, die durch einen gegabelten Abschnitt 76 des beweglichen Bedienelement 36 durchgeführt ist, so daß das bewegliche Bedienelement 36 relativ zu der Schiene 74 beweglich ist. In dem gegabelten Abschnitt 76 des beweglichen Bedienelements 36 ist weiterhin eine Wippe 78 über ein Gelenk 80 verschwenkbar befestigt.

Die Wippe 78 weist eine gekrümmte Abstützung 82 zur Bedienung mit dem Zeigefinger derselben Hand auf, die das Instrument 10 hält. An dem der Abstützung 82 gegenüber liegenden Ende weist die Wippe 78 einen Stift 84 auf, der hinter eine an dem beweglichen Bedienelement 36 befestigte geschwungene Blattfeder 86 greift. Am äußeren freien Ende weist die Blattfeder 86 eine Rolle 88 auf, die mit Zähnen 90 der gezahnten Schiene 74 in und außer Eingriff bringbar ist. Durch Verschwenken der Wippe 78, genauer gesagt der Abstützung 82, nach oben, kommt die durch die Blattfeder 86 nach unten vorgespannte Rolle 88 mit der gezahnten Schiene 74 selbsttätig in Eingriff. Durch Verschwenken der Wippe 78 in umgekehrter Richtung drückt der Stift 84 das freie Ende der Blattfeder 86 nach oben, wodurch die Rolle 88 mit der gezahnten Schiene 74 außer Eingriff kommt.

Mittels der gezahnten Schiene 74 wird es ermöglicht, den distalen Endabschnitt 18 des Rohrschafts 12 in einer Vielzahl von diskreten Winkelstellungen zu arretieren. Wenn die Rolle 88 mit der gezahnten Schiene 74 außer Eingriff gebracht wird, läßt sich der distale Endabschnitt 18 kontinuierlich verschwenken. Die Zähne 90 der gezahnten Schiene 74 sind an ihren Spitzen bevorzugt abgerundet, so daß auch bei mit der gezahnten Schiene 74 in Eingriff stehender Rolle 88 eine möglichst druckfreie Verschwenkung des distalen Endabschnitts 18 in diskreten Abstufungen ermöglicht wird.

Der maximale Winkel + α₂ bzw. - α₁ gemäß Fig. 4 kann durch einen verstellbaren Anschlag, der in Fig. 1 nicht dargestellt ist, und der zwischen dem beweglichen Bedienelement 36 und dem unbeweglichen Bedienelement 38 angeordnet wird, an den verwendeten Lichtwellenleiter 14 angepaßt werden.

Wie aus Fig. 1, 3 und 4 hervorgeht, ist das distale Ende des distalen Endabschnitts 18 abgerundet. Die axiale Länge des distalen Endabschnitts 18 ist bevorzugt nur geringfügig größer als der Außendurchmesser des Rohrschaftes 12. Der Außendurchmesser des distalen Endabschnitts 18 ist bevorzugt gleich dem Außendurchmesser des übrigen Abschnitts des Rohrschafts 12, so daß im Bereich des Übergangs zwischen dem distalen Endabschnitt 18 und dem übrigen Abschnitt des Rohrschafts 12 keine radiale Stufe entsteht.

Der distale Endabschnitt 18 weist ferner eine Fixierhülse 92 auf, so daß die Spitze 16 des Lichtwellenleiters 14 in dem distalen Endabschnitt 18 zumindest radial, aber auch axial fixiert ist. Gemäß Fig. 3 weist der distale Endabschnitt 18 an seinem distalen Ende eine axiale Bohrung 93 auf, in der die Spitze 16 des Lichtwellenleiters 14 in der Fixierhülse 92 im wesentlichen spielfrei fixiert ist.

Gemäß Fig. 1 ist am proximalen Ende des Rohrschafts 12 ein Anschluß 94 zum Verbinden des Rohrschafts 12 mit einer nicht dargestellten Saug- und/oder Spüleinrichtung angeordnet, auf den, wie in Fig. 2 dargestellt ist, ein Stutzen eines Saug- und/oder Spülschlauchs aufsteckbar ist. Die Absaugung bzw. Spülung erfolgt dabei durch das Innere des Innenrohrschaftes 58, in dem auch bei eingesetztem Lichtwellenleiter 14, wie aus Fig. 3 hervorgeht, noch ein Spül- bzw. Saugquerschnitt verbleibt.

Für Absaugung und Spülung können auch zwei separate Anschlüsse vorgesehen werden.

In dem distalen Endabschnitt 18 sind umfänglich verteilt Bohrungen 96 vorgesehen, die als Saug- bzw. Spülöffnungen dienen.

Im folgenden wird nun ein operatives laserchirurgisches Verfahren beschrieben, daß mit dem Instrument 10 durchgeführt werden kann, und zwar am Beispiel der lasergestützten Nasenmuschelbehandlung.

Zunächst wird der Lichtwellenleiter 14 vom proximalen Ende des Rohrschafts 12 her durch einen Einführstutzen 98 eingeführt. Der Rohrstutzen 98 mündet dabei am proximalen Ende 60 des Innenrohrschaftes 58, so daß der Lichtwellenleiter 14 durch den Innenrohrschaft 58 hindurch geschoben werden kann. Der Lichtwellenleiter 14 wird dabei soweit vorgeschoben, bis das lichtaustrittsseitige Ende 16 im distalen Endabschnitt 18 des Rohrschafts 12 zu liegen kommt. Beim Vorschieben den Lichtwellenleiters 14 wird der distale Endabschnitt 18 über das bewegliche Bedienelement 36 in eine mit der Längsachse 22 des Rohrschafts 12 fluchtende 0°-Stellung gebracht. Dadurch wird vermieden, daß die Spitze 16 des Lichtwellenleiters 14 beschädigt wird.

Der Lichtwellenleiter wird mit seinem anderen Ende an einen geeigneten Laser angekoppelt. Die Parameter des Lasers werden entsprechend eingestellt.

Anschließend wird die Spitze 16 des Lichtwellenleiters 14 in das Operationsgebiet geführt und positioniert. Gleichzeitig wird zur Beobachtung des Positioniervorganges und der anschließenden Operation eine Endoskopoptik in das Naseninnere eingeführt. Eine genaue Positionierung der Spitze bzw. des lichtaustrittsseitigen Endes 16 des Lichtwellenleiters 14 und damit der Abstrahlrichtung 24 wird durch Betätigung des beweglichen Bedienelements 36 und durch Drehen des Rohrschafts 12 vorgenommen. Nachdem die Spitze richtig positioniert ist, wird die zu behandelnde Nasenmuschel strichförmig, von hinten nach vorne, bestrahlt. Dies kann dadurch geschehen, daß der distale Endabschnitt 18 und damit die Abstrahlrichtung 24 diskret oder kontinuierlich verschwenkt wird. Während der Behandlung entstehende Rauchgase, die sich durch Vaporisation bzw. Koagulation von Gewebe entwickeln können, werden durch den Innenrohrschaft 58 über die angeschlossene Saugeinrichtung abgesaugt. Zusätzlich kann das Operationsgebiet durch Einleiten einer Spülflüssigkeit durch den Rohrschaft 12 bzw. den Innenrohrschaft 58 gespült werden, um die endoskopische Sichtkontrolle während der Operation nicht zu behindern oder das Operationsgebiet zu kühlen. Die Behandlung des Gewebes kann in dem eingangs genannten Kontakt-Verfahren oder dem Non-Kontakt-Verfahren durchgeführt werden. Es kann auch eine Kombination beider Verfahrenstechniken angewandt werden. Aufgrund der Abwinkelbarkeit der Spitze 16 des Lichtwellenleiters 14 durch Verschwenken des distalen Endabschnitts 18 des Rohrschafts 12 kann die Therapie ohne zwischenzeitliche Entnahme des Instruments 10 aus dem Operationsgebiet mehrfach und an anderen Stellen wiederholt werden.

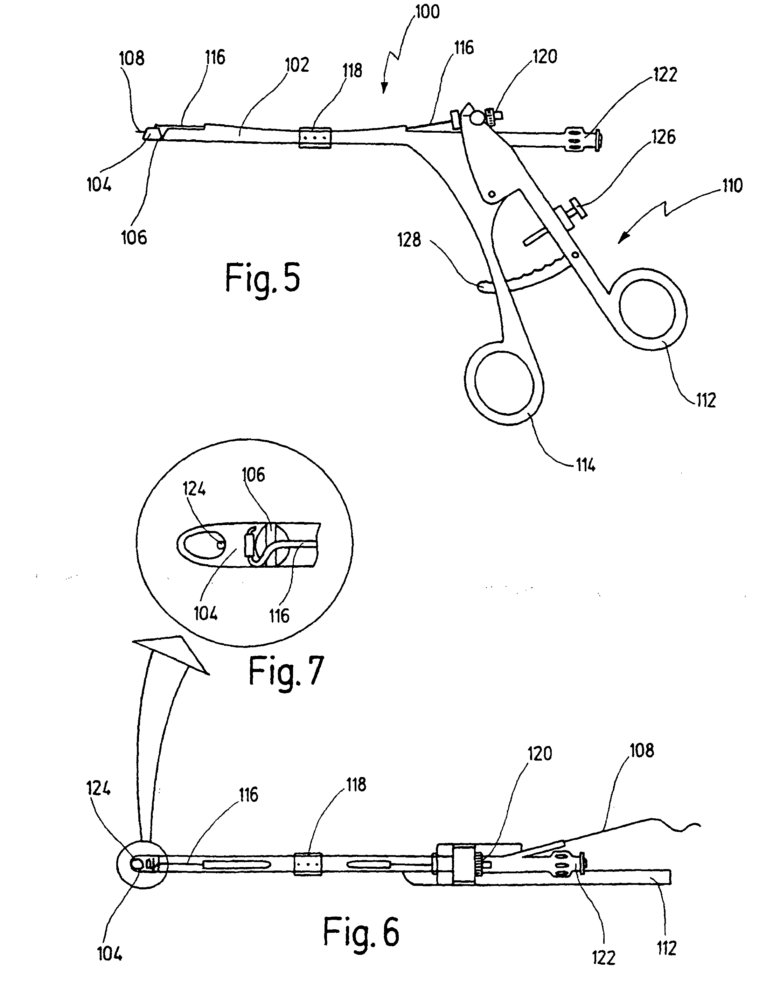

In Fig. 5 bis 7 ist schließlich noch ein weiteres Ausführungsbeispiel eines Instruments 100 dargestellt, das sich von dem Instrument 10 nur geringfügig unterscheidet.

Das Instrument 100 weist demnach wiederum einen zweiteiligen Rohrschaft 102 auf, der einen distalen Endabschnitt 104 aufweist, der mit dem übrigen Abschnitt des Rohrschafts 102 über ein Gelenk 106 in Form eines Scharnieres gelenkig verbunden ist. In den Rohrschaft 102 ist ein Lichtwellenleiter 108 einführbar.

Zum Verschwenken des distalen Endabschnitts 104 weist das Instrument 100 eine Handhabungseinrichtung 110 auf, die ein bewegliches Bedienelement 112 und ein unbewegliches rohrschaftfestes Bedienelement 114 aufweist. Ein Kraftübertragungselement in Form eines drahtförmigen Zug- und Schubelements 116 verbindet das bewegliche Bedienelement 112 kraftschlüssig mit dem distalen Endabschnitt 104 des Rohrschaftes 102. Das Zug- und Schubelement 116 verläuft dabei teilweise durch den Rohrschaft 102.

Der übrige proximale Abschnitt des Rohrschaftes 102 ist starr ausgeführt, weist eine gerade Form oder eine Vorbiegung mit einem Anstellwinkel von ca. 5° auf. Der Rohrschaft 12 weist eine weitere Unterteilung in Form eines drehbaren Kupplungsstückes 118 auf, so daß derjenige Abschnitt des Rohrschafts 102, an dem der distale Endabschnitt 104 verschwenkbar befestigt ist, um die Längsachse des Rohrschafts 102 um 360° gedreht werden kann.

Das Zug- und Schubelement 116 ist weiterhin über eine Spanneinrichtung 120 an dem beweglichen Bedienelement 112 befestigt. Die Spanneinrichtung 120 ermöglicht es, das Zug- und Schubelement 116 mit einer geeigneten Vorspannung mit dem distalen Endabschnitt 104 des Rohrschafts 102 zu verbinden.

Der Rohrschaft 102 ist wiederum als Absaugkanal für Rauch, Applationsprodukte, Gewebe, Flüssigkeiten und als Spülkanal ausgebildet, wozu am proximalen Ende des Rohrschafts 102 ein Anschluß 122 zum Anschließen einer Saug- und/oder Spüleinrichtung vorgesehen ist. Es können auch für Absaugung und Spülung zwei Anschlüsse vorgesehen werden.

Der Lichtwellenleiter 108 ist mit seinem distalen Ende in einer Fixierhülse 124 geführt, die in dem distalen Endabschnitt 102 eingebracht ist.

Eine Begrenzung der Winkelverstellung des distalen Endabschnitts 104 zur Vermeidung eines Überbiegens der Glasfaser bzw. des Lichtwellenleiters 108 wird durch einen verstellbaren Anschlag 126, der am Bedienelement 112 befestigt ist, erreicht.

Eine gerasterte bzw. gezahnte Schiene 128 dient als Arretierung bzw. Verstellsicherung der Winkelstellung des distalen Endabschnitts 104, wodurch die eingestellte Abwinklung des distalen Endabschnitts 104 und damit des distalen Endes des Lichtwellenleiters 108 fixiert werden kann.

Das Instrument 100 unterscheidet sich von dem Instrument 10 somit lediglich durch das Kraftübertragungselement in Form des Zug- und Schubelements 116 und durch die zwischen dem beweglichen Bedienelement 112 und dem unbeweglichen Bedienelement 114 vertauschte Anordnung der Bedienelemente.

## Patentansprüche

1. Instrument zum Applizieren von Licht, insbesondere Laserlicht im menschlichen oder tierischen Körper, insbesondere für die endonasale Laserchirurgie, mit einem Rohrschaft (12; 102), in dessen Inneres ein flexibler Lichtwellenleiter (14; 108) einführbar ist, wobei ein lichtaustrittsseitiges Ende (16) des Lichtwellenleiters (14; 108) in einem distalen Endabschnitt (18; 104) des Rohrschafts (12; 102) zu liegen kommt, wobei der distale Endabschnitt (18; 104) des Rohrschafts (12; 102) über ein scharnierartiges Gelenk (20) mit dem übrigen Abschnitt des Rohrschafts (12; 102) verbunden ist, so daß der distale Endabschnitt (18; 104) aus einer Längsachse (22) des Rohrschafts (12; 102) heraus verschwenkbar ist, und wobei am proximalen Ende des Rohrschafts (12; 102) eine Handhabungseinrichtung (34; 110) zum Verschwenken des distalen Endabschnitts (18; 104) angeordnet ist, die zumindest ein bewegliches Bedienelement (36, 38; 112, 114) aufweist, das über ein axial bewegliches Kraftübertragungselement kraftschlüssig mit dem distalen Endabschnitt (18; 104) verbunden ist, wobei der distale Endabschnitt (18; 104) durch Betätigen des beweglichen Bedienelements (36, 38; 112, 114) in entgegengesetzten Schwenkrichtungen in einem Winkelbereich zwischen einem Winkel (-|α₁|) und einem Winkel (+|α₂|) aus der Längsachse (22) des Rohrschafts (12; 102) heraus verschwenkbar ist.

2. Instrument nach Anspruch 1, wobei der Rohrschaft (12; 102) als Ganzes oder zumindest ein Abschnitt des Rohrschafts (12; 102), an dem der distale Endabschnitt (18; 104) befestigt ist, bezüglich der Handhabungseinrichtung (34; 110) um die Längsachse (22) des Rohrschafts (12; 102) drehbar ist.

3. Instrument nach Anspruch 1 oder 2, wobei der distale Endabschnitt (18; 104) eine axiale Länge aufweist, die nur geringfügig größer als der Außendurchmesser des übrigen Rohrschafts (12; 102) ist.

4. Instrument nach einem der Ansprüche 1 bis 3, wobei der distale Endabschnitt (18; 104) etwa den gleichen Außendurchmesser aufweist wie der übrige Abschnitt des Rohrschafts (12; 102).

5. Instrument nach einem der Ansprüche 1 bis 4, wobei der distale Endabschnitt (18; 104) eine Fixierhülse (92; 124) zum Fixieren des lichtaustrittsseitigen Endes (16) des Lichtwellenleiters (14; 108) aufweist.

6. Instrument nach einem der Ansprüche 1 bis 5, wobei der Rohrschaft (12; 102) einen Anschluß (94; 122) zum Verbinden des Rohrschafts (12; 102) mit einer Saug- und/oder Spüleinrichtung aufweist.

7. Instrument nach Anspruch 6, wobei der bei eingesetztem Lichtwellenleiter (14; 108) verbleibende freie Querschnitt des Innenraums des Rohrschafts (12; 102) gleichzeitig als Saug- und/oder Spülquerschnitt dient.

8. Instrument nach einem der Ansprüche 1 bis 7, wobei der verschwenkbare distale Endabschnitt (18; 104) umfänglich verteilte Öffnungen (96) zum Saugen und/oder Spülen aufweist.

9. Instrument nach einem der Ansprüche 1 bis 8, wobei die Handhabungseinrichtung (34; 110) in Form einer scherengriffartigen Griffanordnung ausgebildet ist.

10. Instrument nach einem der Ansprüche 1 bis 9, wobei das Kraftübertragungselement als Draht oder stabförmiges Zug- und Schubelement (116) ausgebildet ist, das durch das Innere des Rohrschafts (102) verläuft.

11. Instrument nach einem der Ansprüche 1 bis 9, wobei das Kraftübertragungselement als in dem Rohrschaft (12) axial verschieblich angeordneter Innenrohrschaft (58) ausgebildet ist, wobei der Lichtwellenleiter (14) in das Innere des Innenrohrschafts (58) einführbar ist.

12. Instrument nach einem der Ansprüche 1 bis 11, wobei die Verschwenkbarkeit des verschwenkbaren distalen Endabschnitts (18; 104) auf einen vorgegebenen Winkelbereich begrenzt ist.

13. Instrument nach Anspruch 12, wobei die Begrenzung des Verschwenkbereichs über einen verstellbaren Anschlag (126) an der Handhabungseinrichtung einstellbar ist.

14. Instrument nach einem der Ansprüche 1 bis 13, wobei der distale Endabschnitt (18; 104) in einer Vielzahl diskreter oder kontinuierlicher Winkelstellungen bezüglich der Längsachse (22) des Rohrschafts (12; 102) arretierbar ist.

15. Instrument nach Anspruch 14, wobei das zumindest eine bewegliche Bedienelement (36, 38; 112, 114) der Handhabungseinrichtung (34; 110) mit einer eine Vielzahl von Raststellungen aufweisenden gezahnten Schiene (74; 128) in Eingriff bringbar ist.

16. Instrument nach Anspruch 15, wobei Zähne (90) der gezahnten Schiene (74) abgerundet sind.

17. Instrument nach einem der Ansprüche 1 bis 16, wobei das distale Ende des verschwenkbaren distalen Endabschnitts (18) abgerundet ist.

## Claims

1. An instrument for applying light, in particular laser light, in the human or animal body, in particular for endonasal laser surgery, comprising a tubular shaft (12; 102), a flexible light waveguide (14; 108) being introduceable into its interior, wherein a light-emitting end (16) of the waveguide (14; 108) comes to lie in a distal end portion (18; 104) of the tubular shaft (12; 102), wherein the distal end portion (18; 104) of the tubular shaft (12; 102) is connected to the remaining portion of the tubular shaft (12; 102) via a hinge-like joint (20) so that the distal end portion (18; 104) is pivotable out of the longitudinal axis (22) of the tubular shaft (12; 102), and wherein a manipulating device (34; 110) is arranged at the proximal end of the tubular shaft (12; 102) for pivoting the distal end portion (18; 104), which comprises at least one movable operating element (36, 38; 112, 114) being connected with the distal end portion (18; 104) via a force transmission element in force-locking fashion, wherein the distal end portion (18; 104) is pivotable, by actuating the movable operating element (36, 38; 112, 114) in opposite directions of pivoting, in an angle range between an angle (- |α₁|) and an angle + (|α₂|) out of the longitudinal axis (22) of the tubular shaft (12; 102).

2. The instrument of claim 1, wherein the tubular shaft (12; 102) as a whole or at least a portion of the tubular shaft (12; 102) to which the distal end portion (18; 104) is secured is rotatable with respect to the manipulating device (34; 110) about the longitudinal axis (22).

3. The instrument of claims 1 or 2, wherein the distal end portion (18; 104) has an axial length which is only slightly larger than the outer diameter of the remaining tubular shaft (12; 102).

4. The instrument of any one of claims 1 through 3, wherein the distal end portion (18; 104) has about the same outer diameter as the remaining portion of the tubular shaft (12; 102).

5. The instrument of any one of claims 1 through 4, wherein the distal end portion (18; 104) comprises a fixing sleeve (92; 124) for fixing the light-emitting end (16) of the waveguide (14; 108).

6. The instrument of any one of claims 1 through 5, wherein the tubular shaft (12; 102) comprises a connector (94; 122) for connecting the tubular shaft (12; 102) with a suctioning and/or irrigating device.

7. The instrument of claim 6, wherein the remaining free cross-section of the tubular shaft (12; 102) when the waveguide is inserted serves as suctioning and/or irrigating passage.

8. The instrument of any one of claims 1 through 7, wherein the pivotable distal end portion (18; 104) comprises openings (96) distributed about its circumference for suctioning and/or irrigating.

9. The instrument of any one of claims 1 through 8, wherein the manipulating device (34; 110) is configured in the form of a scissors-like handle arrangement.

10. The instrument of any one of claims 1 through 9, wherein the force transmission element is configured as a wire or rod-like push and pull element (116), which runs through the interior of the tubular shaft (102).

11. The instrument of any one of claims 1 through 9, wherein the force transmission element is formed as an inner tubular shaft (58) arranged to be axially slidable within the tubular shaft (12) with the waveguide (14) being introduceable in the interior of the inner tubular shaft (58).

12. The instrument of any one of claims 1 through 11, wherein the pivotal movability of the pivotable distal end portion (18; 104) is limited to a predetermined angular range.

13. The instrument of claim 12, wherein the limit of the pivotal range is adjustable by an adjustable stop (126) on the manipulating device.

14. The instrument of any one of claims 1 through 13, wherein the distal end portion (18; 104) can be locked in a plurality of discrete or continuous angular positions with respect to the longitudinal axis (22) of the tubular shaft (12; 102).

15. The instrument of claim 14, wherein the at least one movable operating element (36, 38; 112, 114) of the manipulating device (34; 110) is engageable with a toothed rod (74; 128) having a plurality of locking positions.

16. The instrument of claim 15, wherein the teeth (90) of the toothed rod (74) are rounded.

17. The instrument of any one of claims 1 through 16, wherein the distal end of the pivotable distal end portion (18) is rounded.

## Revendications

1. Instrument pour appliquer de la lumière, plus particulièrement de la lumière laser, dans le corps humain ou dans le corps d'animaux, notamment pour la chirurgie laser endonasale, avec un manchon tubulaire (12 ; 102) à l'intérieur duquel un guide d'ondes lumineuses flexible (14 ; 108) peut être inséré, une extrémité du côté de la sortie de la lumière (16) du guide d'ondes lumineuses (14 ; 108) étant en contact avec une partie d'extrémité distale (18 ; 104) du manchon tubulaire (12; 102), la partie d'extrémité distale (18 ; 104) du manchon (12; 102) étant reliée à la partie restante du manchon tubulaire (12; 102) par l'intermédiaire d'une articulation en forme de charnière (20), de telle sorte que la partie d'extrémité distale (18; 104) peut être pivotée hors d'un axe longitudinal (22) du manchon tubulaire (12; 102), et un dispositif de manipulation (34 ; 110) pour pivoter la partie d'extrémité distale (18 ; 104) se trouvant au niveau de l'extrémité proximale du manchon tubulaire (12; 102), qui comprend au moins un élément mobile (36, 38 ; 112, 114) relié, avec une continuité de force, par l'intermédiaire d'un élément de transmission de force, à la partie d'extrémité distale (18 ; 104), la partie d'extrémité distale (18; 104) pouvant être pivotée, grâce à l'actionnement de l'élément mobile (36, 38 ; 112, 114) dans des directions de pivotement opposées dans un domaine angulaire entre un angle (-|α₁|) et un angle (+|α₂|) hors de l'axe longitudinal (22) du manchon tubulaire (12 ; 102).

2. Instrument selon la revendication 1, le manchon tubulaire (12; 102) dans son ensemble ou au moins une partie du manchon tubulaire (12 ; 102) à laquelle la partie d'extrémité distale (18; 104) est fixée, étant rotatif par rapport au dispositif de manipulation (34; 110) autour de l'axe longitudinal (22) du manchon tubulaire (12 ; 102).

3. Instrument selon la revendication 1 ou 2, la partie d'extrémité distale (18 ; 104) comprenant une longueur axiale qui n'est que légèrement plus importante que le diamètre extérieur du reste du manchon tubulaire (12 ; 102).

4. Instrument selon l'une des revendications 1 à 3, la partie d'extrémité distale (18; 104) présentant approximativement le même diamètre extérieur que la partie restante du manchon tubulaire (12 ; 102).

5. Instrument selon l'une des revendications 1 à 4, la partie d'extrémité distale (18 ; 104) comprenant une douille de fixation (92 ; 124) pour la fixation de l'extrémité du côté de la sortie de la lumière (16) du guide d'ondes lumineuses (14 ; 108).

6. Instrument selon l'une des revendications 1 à 5, le manchon tubulaire (12 ; 102) comprenant un raccord (94 ; 122) pour la liaison du manchon tubulaire (12; 102) avec un dispositif d'aspiration et/ou de nettoyage.

7. Instrument selon la revendication 6, la section libre restante de l'intérieur du manchon tubulaire (12; 102) lorsque le guide d'ondes lumineuses (14; 108) est inséré servant en même temps de section d'aspiration et/ou de nettoyage.

8. Instrument selon l'une des revendications 1 à 7, la partie d'extrémité distale pivotante (18; 104) comprenant des ouvertures (96) réparties sur sa circonférence pour l'aspiration et le nettoyage.

9. Instrument selon l'une des revendications 1 à 8, le dispositif de manipulation (34; 110) se présentant sous la forme d'un dispositif à poignée.

10. Instrument selon l'une des revendications 1 à 9, l'élément de transmission de la force étant conçu comme un fil ou un élément de traction et de poussée en forme de tige (116), qui passe à l'intérieur du manchon tubulaire (102).

11. Instrument selon l'une des revendications 1 à 9, l'élément de transmission de la force étant conçu comme un manchon tubulaire interne (58) disposé de manière mobile axialement dans le manchon tubulaire (12), le guide d'ondes lumineuses (14) pouvant être inséré à l'intérieur du manchon tubulaire interne (58).

12. Instrument selon l'une des revendications 1 à 11, la possibilité de pivotement de la partie d'extrémité distale pivotante (18; 104) étant limitée à un domaine angulaire prédéterminé.

13. Instrument selon la revendication 12, la limitation du domaine de pivotement pouvant être réglée par l'intermédiaire d'une butée réglable (126) au niveau du dispositif de manipulation.

14. Instrument selon l'une des revendications 1 à 13, la partie d'extrémité distale (18 ; 104) pouvant être bloquée dans une pluralité de positions angulaires continues ou discrètes par rapport à l'axe longitudinal (22) du manchon tubulaire (12 ; 102).

15. Instrument selon la revendication 14, l'élément mobile (36, 38 ; 112; 114) du dispositif de manipulation (34 ; 110) pouvant être emboîté avec une glissière dentée (74 ; 128) comprenant une pluralité de positions d'encliquetage.

16. Instrument selon la revendication 15, les dents (90) de la glissière dentée (74) étant arrondies.

17. Instrument selon l'une des revendications 1 à 16, l'extrémité distale de la partie d'extrémité distale pivotante (18) étant arrondie.
